# EUROPÄISCHE PATENTANMELDUNG

(11) **EP 1 745 796 A1**
(43) Veröffentlichungstag der Anmeldung: **24.01.2007**
(21) Anmeldenummer: 05015692.6
(22) Anmeldetag: 19.07.2005
(51) Int. Cl.: A61K 36/71, A61K 36/53, A61K 36/85, A61K 36/28, A61K 31/566, A61K 31/357, A61K 31/352, A61P 35/00, A23L 1/30

(54) **Kombinationspräparat insbesondere zur Therapie des Prostatakarzinoms**

(71) Anmelder: Bionorica Research GmbH, 6020 Innsbruck (AT)
(72) Erfinder: Popp, Michael A., 91207 Lauf/Pegnitz (DE); Seidlova-Wuttke, Dana, 37120 Bovenden (DE); Wuttke, Wolfgang, 37120 Bovenden (DE); Jarry, Hubertus, 37249 Neu-Eichenberg (DE)
(74) Vertreter: Vossius & Partner

(57) **Zusammenfassung**

Die Erfindung betrifft eine Zusammensetzung aus Komponenten zur gleichzeitigen oder sequentiellen Verabreichung oder Einnahme, wobei die Komponenten einen 5α-Reduktase Hemmstoff, einen Östrogenrezeptor β-Agonisten sowie einen Hemmstoff des prostataspezifischen Antigens umfassen. Die Erfindung betrifft auch die Verwendung eines 5α-Reduktase Hemmstoff, eines Östrogenrezeptor β-Agonisten und eines Hemmstoffes des prostataspezifischen Antigens zur Herstellung eines Arzneimittels zur Prävention oder Behandlung einer Prostataerkrankung. Des weiteren betrifft die Erfindung die Verwendung eines 5α-Reduktase Hemmstoffes, eines Östrogenrezeptor β-Agonisten und eines Hemmstoffes des prostataspezifischen Antigens zur Herstellung eines Nahrungsergänzungsmittels zur Prävention einer Prostataerkrankung. In bevorzugten Ausführungsformen der erfindungsmäßigen Zusammensetzung bzw. Verwendung werden als 5α-Reduktase Hemmstoff ein Extrakt aus Cimifuga racemosa, ein Extrakt aus Vitex agnus castus oder aus Silybum marianum als Östrogenrezeptor β-Agonist und ein Extrakt aus Belamcanda chinensis als Hemmstoff des prostataspezifischen Antigens eingesetzt.

## Beschreibung

Die Erfindung betrifft eine Zusammensetzung aus Komponenten zur gleichzeitigen oder sequentiellen Verabreichung oder Einnahme, wobei die Komponenten einen 5α-Reduktase Hemmstoff, einen Östrogenrezeptor β-Agonisten sowie einen Hemmstoff des prostataspezifischen Antigens umfassen. Die Erfindung betrifft auch die Verwendung eines 5α-Reduktase Hemmstoffes, eines Östrogenrezeptor β-Agonisten und eines Hemmstoffes des prostataspezifischen Antigens zur Herstellung eines Arzneimittels zur Prävention oder Behandlung einer Prostataerkrankung. Des weiteren betrifft die Erfindung die Verwendung eines 5α-Reduktase Hemmstoffes, eines Östrogenrezeptor β-Agonisten und eines Hemmstoffes des prostataspezifischen Antigens zur Herstellung eines Nahrungsergänzungsmittels zur Prävention einer Prostataerkrankung. In bevorzugten Ausführungsformen der erfindungsmäßigen Zusammensetzung bzw. Verwendung werden als 5α-Reduktase Hemmstoff ein Extrakt aus Cimifuga racemosa, ein Extrakt aus Vitex agnus castus oder aus Silybum marianum als Östrogenrezeptor β-Agonist und ein Extrakt aus Belamcanda chinensis als Hemmstoff des prostataspezifischen Antigens eingesetzt.

In der Beschreibung werden eine Reihe an Dokumenten inklusive Patentanmeldungen und Hersteller-Gebrauchsanweisungen aus dem Stand der Technik genannt. Während der Offenbarungsgehalt dieser Dokumente als nicht relevant für die Patentfähigkeit der Erfindung angesehen wird, ist er per Referenz in die vorliegende Beschreibung inkorporiert.

Häufig auftretende Prostataerkrankungen schließen maligne und benigne Prostatahyperplasien ein. Insbesondere für die maligne Prostatahyperplasie (Prostatakarzinom) ergeben sich aus heutiger Sicht neben operativen Eingriffen, Bestrahlung und der Chemotherapie wenig wirksame Alternativen zur hormonablativen Therapie durch GnRH-Analoga bzw. Kastration.

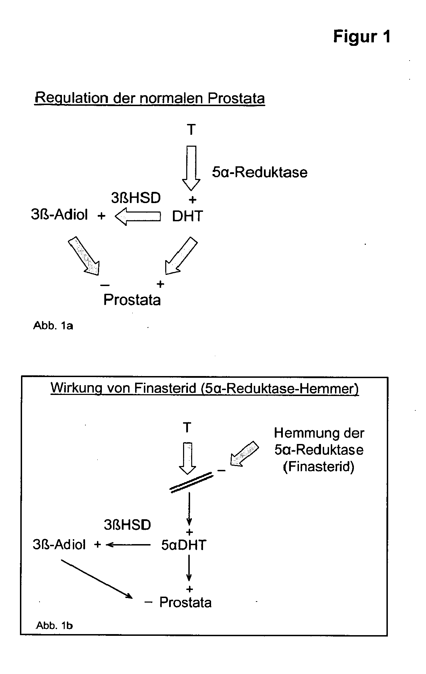

In jüngster Zeit hat eine relativ "organspezifische" hormonablative Therapie gezeigt, dass sie die Zahl auftretender Prostatakarzinome (PCa) signifikant reduzieren kann, also präventiv einsetzbar ist. Es handelt sich dabei um die Therapie mit dem 5α-Reduktasehemmer Finasterid. Dieses Enzym wandelt das im Blut zirkulierende Testosteron, welches in großen Mengen von den Hoden produziert wird, in 5α-Dihydrotestosteron (5αDHT) um, welches das eigentlich wirksame Androgen in der Prostata ist. Das Enzym, welches Testosteron zu 5αDHT reduziert, ist die 5α-Reduktase (vgl. Figur 1). Von diesem Enzym gibt es zwei auf unterschiedlichen Genen codierte Formen. Die 5α-Reduktase-2 ist die überwiegend in Prostata und Samenblase wirksame Form, während die 5α-Reduktase-1 überwiegend in Haarfollikeln, Talgdrüsen und anderen epithelialen Strukturen wirksam ist.

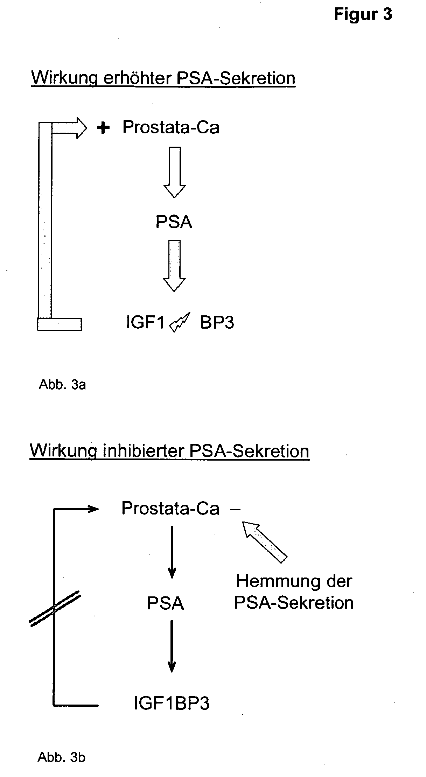

Ein weiterer neuer therapeutischer Ansatz ergibt sich aus der Kenntnis der biologischen Wirksamkeit des prostataspezifischen Antigens (PSA). Bei dem PSA handelt es sich um ein Enzym, welches Proteaseaktivität besitzt und in der Prostata das *insulin like growth factor-1* bindende Protein (IGF1 BP3) zu spalten vermag, so dass IGF1 nicht mehr gebunden und damit bioneutralisiert werden kann (s. Figur 3). Der Wachstumsfaktor IGF1 hat in allen bisher getesteten Geweben inklusive PCa- und BPH-Gewebe eine proliferationsfördernde Rolle, stimuliert also das Wachstum von BPH und PCa. Das Enzym PSA wird etwa proportional zum Prostatagewicht und besonders viel in karzinomatös entartetem Gewebe wiederum proportional zum Prostatakarzinomgewicht produziert. Es kommt also bei großen Tumoren zu hohen PSA-Spiegeln, und diese Erhöhung ist in der Regel auch im Blut nachweisbar. In der Prostata bewirkt die vermehrte PSA-Produktion eine Hemmung der Bindung von IGF1, da IGF1BP3 durch PSA inaktiviert wird. Dadurch steht mehr freies IGF1 zur Verfügung, welches zu vermehrter und rascherer Proliferation des Tumors führt, und damit kommt es zu einem deletären Circulus vitiosus.

Auf der Basis der Erkenntnis der Expression der oben genannten Gene und der Funktion der entsprechenden Proteine in der Entwicklung von Prostataerkrankungen sind in der Vergangenheit eine Reihe von Ansätzen entwickelt worden, die der Bekämpfung dieser Krankheiten und insbesondere des Prostatakarzinoms dienen. Kennzeichnend für die überwiegende Mehrzahl dieser Ansätze ist ein Einsatz einer einzelnen pharmakologischen Wirksubstanz oder, bei Phytopharmaka, einer einzigen Pflanze als Quelle der pharmakologisch wirksamen Substanz(en). Diese Art Ansatz wird im Stand der Technik auch als Monotherapie beschrieben. So wird in der DE 101 46 159 A1 dargelegt, dass Extrakte aus Cimicifuga-Arten zur Behandlung von benignen und malignen Prostatahyperplasien eingesetzt werden können. In der DE 101 23 503 A1 werden Ansätze zur Behandlung von benignen und malignen Prostatahyperplasien mittels Extrakten aus Belamcanda chinensis vorgestellt. In einer kürzlich veröffentlichten Zusammenfassung von Studien über die Verwendung transgener Tiere zur Aufklärung der Ätiologie von Prostatakarzinomen wurde festgehalten, dass es zur Zeit kein geeignetes Maussystem gibt, dass den Ausbruch und die Metastasierung dieser Krebsart erklären kann (Kasper und Smith, J. Urology 172 (2004), 12-19). Eine Ursache dafür wurde von den Autoren darin gesehen, dass in den Mausmodellen jeweils nur die fehlende oder die Überexpression eines in der Ätiologie des Prostatakarzinoms vermuteten Gens untersucht wurde.

Bei der sich abzeichnenden Komplexität der Interaktionen zwischen verschiedenen Genprodukten in der Ätiologie von Prostataerkrankungen und insbesondere von Prostatakrebs erscheint allerdings die Entwicklung von neuartigen Therapieansätzen wünschenswert, die an verschiedenen Knotenpunkten dieser Interaktionen angreifen und somit wirksamere Therapieansätze der genannten Erkrankungen versprechen. Das der Erfindung zugrunde liegende technische Problem bestand dem gemäß darin, derartige neue Therapieansätze bereitzustellen. Dieses technische Problem wird durch die in den Patentansprüchen bereit gestellten Ausführungsformen gelöst.

Somit betrifft die Erfindung eine Zusammensetzung aus Komponenten zur gleichzeitigen oder sequentiellen Verabreichung oder Einnahme, wobei die Komponenten einen 5α-Reduktase Hemmstoff, einen Östrogenrezeptor β-Agonisten sowie einen Hemmstoff des prostataspezifischen Antigens umfassen.

Der Begriff "Komponente" bezeichnet erfindungsgemäß eine Monosubstanz (ggf. suspendiert, dispergiert oder in Lösung etc.) oder ein Substanzgemisch (ebenfalls ggf. in Lösung oder suspendiert, dispergiert etc.) das eine oder mehrere Substanzen enthält, die die gewünschte Eigenschaften aufweist/aufweisen oder dieser entspricht/diesen entsprechen.

Der Begriff "gleichzeitige oder sequentielle Verabreichung" bedeutet im Sinne dieser Erfindung, dass die drei aktiven Substanzen entweder zusammen (gleichzeitig) und zwar vorzugsweise in einer Dosis oder nacheinander (sequentiell) in mehreren Dosen verabreicht werden wobei auch bei der sequentiellen Verabreichung darauf zu achten ist, dass die Wirkkomponenten ihre pharmakologische / therapeutische / präventive Aktivität gleichzeitig oder im Wesentlichen gleichzeitig ausüben. Bei der gleichzeitigen wie auch der sequentiellen Verabreichung können prinzipiell verschiedene Verabreichungswege beschritten werden. Bei der sequentiellen Verabreichung trifft dies auch für die Verabreichung der einzelnen Dosen zu. Allerdings ist sowohl bei der gleichzeitigen wie auch bei der sequentiellen Verabreichung (für sämtliche Dosen) der orale Verabreichungs- bzw. Einnahmeweg bevorzugt.

Der Begriff "5α-Reduktase Hemmstoff" ist im Stand der Technik bekannt. Er bezeichnet Substanzen (wobei diese Substanzen auch in Substanzgemischen vorkommen können), die die Aktivität der 5α-Reduktase unter physiologischen Bedingungen hemmen. Unter "Hemmung" wird dabei erfindungsgemäß (auch im Zusammenhang mit weiteren Ausführungsformen der Erfindung) verstanden, dass die Aktivität des Enzyms um mindestens 50%, vorzugsweise mindestens 75%, stärker bevorzugt mindestens 90% und besonders bevorzugt mindestens 95% gemindert wird. Idealerweise wird das Enzym in seiner Aktivität um mindestens 98%, z.B. mindestens 99% gehemmt. Unter der Aktivität des Enzyms wird hier die Umsetzung von Testosteron in 5α-Testosteron verstanden. Geeignete Readout-Systeme zur Messung der Umsetzung schließen die Messung des Ausgangssubstrats sowie des Endproduktes unter Einschluss einer geeigneten Kontrolle, z.B. eines Versuchsansatzes ohne die Zugabe an aktivem Enzym ein.

Ein im Stand der Technik bekanntes Beispiel für einen derartigen Hemmstoff ist Finasterid (Freiname für *N-tert*-Butyl-3-oxo-4-aza-5α-androst-1-en-17β-carboxamid). Weitere bekannte 5α-Reduktase Hemmstoffe, die als Komponenten der erfindungsgemäßen Zusammensetzung ins Auge gefasst werden, sind Dutasterid und Epristerid. Als Quelle weiterer Hemmstoffe sind Extrakte von Cimicifuga racemosa bekannt. Der Fachmann ist darüber hinaus in der Lage, auf der Basis seines Fachwissens weitere Hemmstoffe mit einer derartigen Aktivität zu isolieren. Beispielsweise können Bibliotheken an niedermolekularen Substanzen organischen oder anorganischen Ursprungs, vorzugsweise im Hochdurchsatz-Screening, auf die Umsetzung von Testosteron in 5α-Testosteron durchsucht werden.

Geeignete Ansätze für das Hochdurchsatz-Screening sind publiziert. Screening-Assays werden in der Regel in flüssiger Phase durchgeführt, wobei für jede zu testende Verbindung mindestens ein Reaktionsansatz gemacht wird. Als Behälter kommen typischerweise Mikrotiterplatten zum Einsatz, die beispielsweise 96, 384 oder 1536 Reaktionsgefäße aufweisen. Roboter, Bandstrassensysteme und computergestützte Steuerung sind weitere, dem Fachmann geläufige und häufig zum Einsatz kommende Bestandteile einer Einrichtung zum Hochdurchsatz-Screening. Der Assay kann als "Eintopf"-Reaktion ausgelegt werden, was in der Regel, sofern durchführbar, zu bevorzugen ist, kann jedoch auch Wasch- und/oder Transferschritte umfassen. Detektionsschemata beruhen beispielsweise auf Radioaktivität, Lumineszenz oder Fluoreszenz, wie etwa Fluoreszenz-Resonanz-Energietransfer (FRET) und Fluoreszenz-Polarisation (FP). Hinsichtlich des im Rahmen des Assays zum Einsatz kommenden biologischen Materials sind insbesondere biochemische Assays, zelluläre Assays und in vivo Assays zu erwähnen. Während biochemische Assays, in denen das Zielmolekül allein, d.h. außerhalb des zellulären Kontexts vorliegt, in der Regel direkte Rückschlüsse zulassen, zeichnen sich zelluläre Assays durch ihre Breite aus, d.h. die Möglichkeit, mit einer ganzen Reihe zellulärer Mechanismen zu interferieren, solange ein gewünschter Phänotyp oder Readout erzielt wird. Für die in vivo Assays kommen geeignete Tiermodelle, wie etwa die Maus (siehe die beigefügten Beispiele) zum Einsatz. Die Durchforstung von Substanzbibliotheken mit einigen Hunderttausend Substanzen nimmt in der Regel zwischen Tagen und Wochen in Anspruch. Ein experimenteller Hochdurchsatz-Screen kann durch einen virtuellen Screen ergänzt oder gegebenenfalls sogar ersetzt werden. Virtuelle oder computer-basierte Screening-Ansätze sind auf strukturelle Informationen angewiesen. Ist die Struktur des Target-Moleküls bekannt, so kommen Verfahren zum Einsatz, die unter dem Begriff "Docking" subsumiert werden. Sind hingegen mehrere, an das Targetmolekül bindende Substanzen strukturell bekannt, so können Verfahren zum Pharmacophor-Modelling verwendet werden, mit dem Ziel neue, ebenfalls bindende Substanzen zu entwerfen.

Als Readout-System kommt ein für das 5α-Testosteron spezifischer Antikörper in Frage, der Testosteron nicht erkennt. Die Bindung derartiger Antikörper an das 5α-Testosteron kann beispielsweise über eine Markierung des Antikörpers nachgewiesen werden. Verschiedene Ansätze zum Nachweis immunologischer Reagenzien wie Antikörper sind z.B. in Harlow und Lane, "Antibodies, A Laboratory Manual", CSH Press 1988, Cold Spring Harbor, beschrieben. Eine einmal aufgefundene Substanz kann mit üblichen Verfahren von einer Leitsubstanz in eine geeignete pharmakologische Substanz weiterentwickelt werden. Alternativ dazu kann die einmal isolierte Substanz direkten Eingang in die erfindungsgemäße Zusammensetzung finden. Im Falle eines im Tiermodell durchgeführten Screens stellt das Tumorwachstum bzw. dessen Verzögerung, Unterdrückung oder die partielle oder vollständige Remission des Tumors einen geeigneten Assay-Readout dar.

Wie weiter oben dargestellt, sind auf zwei auf unterschiedlichen Genen codierte Formen der 5α-Reduktase bekannt, wobei die 5α-Reduktase-2 (auch als 5α-Reduktase Typ 2 bezeichnet) überwiegend in Prostata und Samenblase wirksam ist, und die 5α-Reduktase-1 (auch als 5α-Reduktase Typ 1 bezeichnet) überwiegend in Haarfollikeln, Talgdrüsen und anderen epithelialen Strukturen wirksam ist. Der erfindungsgemäße 5α-Reduktase Hemmstoff hemmt 5α-Reduktase-2 und/oder 5α-Reduktase-1. In einer besonders bevorzugten Ausführungsform ist der erfindungsgemäße 5α-Reduktase Hemmstoff ein 5α-Reduktase-2 Hemmstoff.

Der Begriff "Östrogenrezeptor β-Agonist" ist im Stand der Technik ebenfalls bekannt. Er bezeichnet eine Substanz (die ebenfalls in einem Substanzgemisch vorkommen kann), die die Aktivität des Östrogenrezeptors vom β-Subtyp (ERβ) aktiviert. Dieser Rezeptor wurde vor einigen Jahren von der Arbeitsgruppe um Gustafsson als zweiter Östrogenrezeptor (ER) kloniert (Kuiper, G. G. J. M. et al., Endocrinology 139, 4252-4263 (1998)). Interessanterweise wurde dieser ERβ (der alte, schon lange bekannte ist der ERα) auf der Suche nach einem weiteren Androgenrezeptor (AR) aus Prostatagewebe kloniert. ERβ wird in hohen Konzentrationen in der Prostata exprimiert und der endogene Ligand des ERβ in der Prostata soll das 5α-Androstan-3β,17β-diol (3βAdiol) sein, welches ein Metabolit des 5αDHT ist. Dem 3βAdiol wird eine 5αDHT-antagonistische Wirkung zugeschrieben. 5αDHT stimuliert also das Prostatawachstum, so auch das Wachstum von Prostatakarzinomen, während 3βAdiol proliferationshemmend wirken soll. Durch die Aktivierung von ERβ in Prostata bzw. im Karzinomgewebe ist die Proliferation hemmbar und der programmierte Zelltod (Apoptose) stimulierbar. Die Aktivierung des ERβ vermittels der in der erfindungsgemäßen Zusammensetzung befindlichen Komponente beträgt mindestens 30%, vorzugsweise mindestens 50%, stärker bevorzugt mindestens 75% und besonders bevorzugt mindestens 100% im Vergleich zu einer geeigneten Negativkontrolle (beispielsweise eines Versuchsansatzes, in dem die Komponente weggelassen wird). Idealerweise beträgt der Grad der Aktivierung mindestens 200% wie mindestens 500% oder auch mindestens 1000% im Vergleich zur Negativkontrolle. Beispiele geeigneter, im Stand der Technik bekannter Östrogenrezeptor β-Agonisten sind das Apigenin aus der Pflanze Vitex agnus castus und das Silybinin aus der Pflanze Silybum marianum. Auch im Falle dieser Art von Hemmstoffen ist der Fachmann in der Lage, weitere geeignete Substanzen, beispielsweise aus Bibliotheken niedermolekularer Substanzen zu isolieren. Ein geeigneter Test zur Auffindung von Östrogenrezeptor β-Agonisten ist ein Bindungsassay unter Verwendung von rekombinantem ERβ-Protein als Zielmolekül. Geeignete Detektionsschemata sind weiter oben im Zusammenhang mit der Beschreibung von Hochdurchsatz-Assays zur Auffindung von Reduktasehemmern dargestellt, die mutatis mutandis hier Anwendung finden können. Zusätzlich oder alternativ kann die Aktivierung von ERβ durch einen erfindungsgemäßen Östrogenrezeptor β-Agonisten in zellulären oder in vivo Assays festgestellt werden. Hierzu können mit einem Reportgen transfizierte Zellen zum Einsatz gelangen, wobei das Reportgen in Abhängigkeit des Aktivierungszustands von ERβ exprimiert wird. Weitere geeignete Tests für die Isolierung bzw. Testung der vorstehend genannten Hemmstoffe können auf der Basis der Lehre der WO99/47149 dargestellt werden.

Der Begriff "Hemmstoff des prostataspezifischen Antigens" ist ebenfalls im Stand der Technik bekannt und bezeichnet Substanzen, die die enzymatische Aktivität von PSA inhibieren. Im Stand der Technik bekannte Hemmstoffe schließen die Isoflavine wie Tectorigenin ein. Neue Hemmstoffe des prostataspezifischen Antigens können ebenfalls aus Bibliotheken niedermolekularer Substanzen abgeleitet werden (zum Prinzip siehe oben). Die Hemmung der PSA-Sekretion durch Zusetzen von Hemmstoffen kann in vitro in den Kulturüberständen geeigneter Zellen, beispielsweise LNCaP-Zellen, gemessen werden. LNCaP-Zellen sind PSAproduzierende Zellen, die von einem humanen Prostata-Karzinom stammen. Die ATCC-Hinterlegungsnummer von LNCaP-Zellen ist CRL-1740.

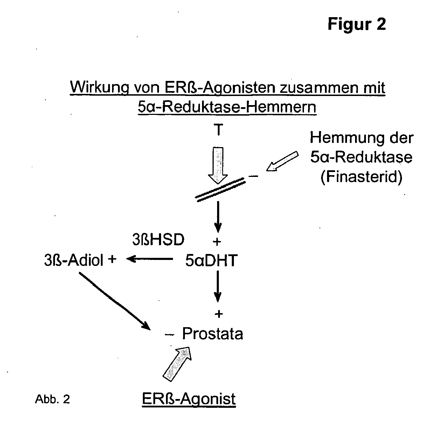

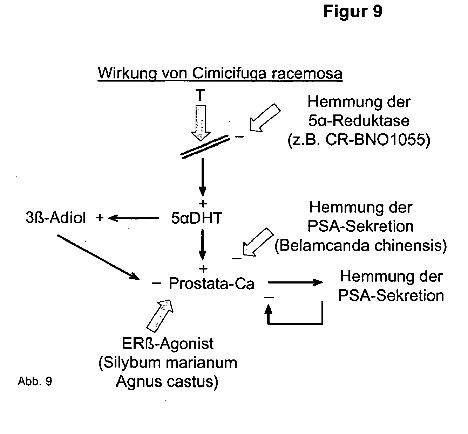

Die erfindungsgemäße Lösung des oben genannten technischen Problems beschreitet insofern neue Wege, als über eine Kombination an Komponenten, die jede für sich an einem anderen kritischen Punkt in der Ätiologie der genannten Prostataerkrankungen angreifen, ein therapeutischer Effekt erzielt wird, der dem auf der Basis einer Monosubstanz weit überlegen ist (s. schematische Darstellung in Fig. 9). Eine Therapie mit der erfindungsgemäßen Zusammensetzung hemmt die 5α-Reduktase, der nachteilige Einfluss der Hemmung der Bildung von 3βAdiol wird durch gleichzeitige Gabe des ERβ-Agonisten kompensiert (vgl. Fig. 2). Die (im Wesentlichen) zeitgleiche Therapie mit einem PSA Hemmstoff hat besonders starke hemmende Wirkungen auf die PSA-Sekretion und inhibiert damit die Bioverfügbarkeit von IGF1 und damit den proliferationsstimulierenden Effekt dieses Wachstumsfaktors. Der PSA-Hemmstoff wird ferner die Hemmung der Sekretion von VEGF und damit eine Hemmung von Gefäßneubildung bewirken. Der vorstehend im Zusammenhang mit der PSA-Expression genannte Circulus vitiosus wird erfindungsgemäß damit durch Hemmung der PSA-Sekretion durchbrochen. Diese Erkenntnisse stellen insgesamt einen völlig neuen therapeutischen Ansatz zur Therapie von Erkrankungen der Prostata und insbesondere der benignen Prostatahyperplasie wie auch des Prostatakarzinoms dar (vgl. Figur 9).

In einer bevorzugten Ausführungsform der erfindungsmäßigen Zusammensetzung wird als 5α-Reduktase Hemmstoff ein Extrakt aus Cimifuga racemosa eingesetzt.

Extrakte aus dieser Pflanze können aus den verschiedenen Bestandteilen der Pflanze hergestellt werden, wobei bevorzugt Rhizome, Blätter, Wurzeln, Stengel und/oder Blütenblätter eingesetzt werden. Bevorzugte Extraktionsmittel sind überkritisches CO₂ und Gemische von Wasser mit organischen Lösungsmitteln. In einer besonders bevorzugten Ausführungsform der Erfindung ist der Extrakt aus Cimicifuga racemosa ein wässrig-ethanolischer Extrakt aus Cimicifugawurzelstock. Ein Beispiel, wie ein derartiger Extrakt von Cimicifuga racemosa hergestellt werden kann, ist in der WO99/47149 beschrieben. Ein Beispiel eines wässrig-ethanolischer Extrakt aus Cimicifugawurzelstock ist der hier als CR BNO 1055 bezeichnete Extrakt.

In einer anderen bevorzugten Ausführungsform betrifft die Erfindung eine Zusammensetzung, wobei als 5α-Reduktase Hemmstoff Finasterid eingesetzt wird. Finasterid und seine pharmaologischen Eigenschaften sind im Stand der Technik bekannt. Nicht bekannt waren die vorteilhaften Eigenschaften in Kombination mit den weiteren Komponenten der erfindungsgemäßen Zusammensetzung zur Behandlung der genannten Prostataerkrankungen.

In einer weiteren bevorzugten Ausführungsform der erfindungsgemäßen Zusammensetzung wird ein Extrakt aus Vitex agnus castus oder aus Silybum marianum als Quelle eines Östrogenrezeptor β-Agonisten eingesetzt. In der Vergangenheit wurde gezeigt, dass Extrakte aus diesen Pflanzenarten die Aktivität eines ERβ-Agonisten aufweisen. In der Folge konnte als ERβ-Agonist aus Vitex agnus castus das Apigenin (Jarry, H. et al., Planta Med. 69, 945-947 (2003)) isoliert werden und aus Silybum marianum das Silybinin (Seidlova-Wuttke, D. et al., Journal of Steroid Biochemistry & Molecular Biology 86, 179-188 (2003)). Als Basis für die erfindungsgemäßen Extrakte können im wesentlichen alle Bestandteile der jeweiligen Pflanze eingesetzt werden. Dazu gehören Rinde, Äste, Samen, Früchte und Stammholz. Bevorzugt werden zur Herstellung der Extrakte Rhizome, Stengel, Blätter und/oder Blütenblätter der Pflanzen verwendet. Bevorzugte zur Extraktion verwendete Lösungsmittel sind überkritisches CO₂ und Gemische von Wasser mit organischen Lösungsmitteln.

Bevorzugt ist im Sinne dieser Erfindung daher auch eine Zusammensetzung, in der als Östrogen rezeptor β-Agonist Silybinin oder Apigenin eingesetzt wird. Diese Substanzen können für den Einsatz in der erfindungsgemäßen Zusammensetzung aus den Extrakten der vorgenannten Pflanzen isoliert werden. Alternativ können diese Substanzen, deren Struktur bekannt ist, mit den Mitteln der organischchemischen Synthese hergestellt werden.

Bevorzugt ist erfindungsgemäß ferner eine Ausführungsform, in der für die Zusammensetzung ein Extrakt aus Belamcanda chinensis als Hemmstoff bzw. als Quelle eines Hemmstoffs des prostataspezifischen Antigens eingesetzt wird. Als Basis für den Extrakt können im wesentlichen alle Bestandteile der Pflanze eingesetzt werden. Dazu gehören Rinde, Äste, Samen, Früchte und Stammholz. Bevorzugt werden zur Herstellung der Extrakte Rhizome, Stengel, Blätter und/oder Blütenblätter der Pflanzen verwendet. Bevorzugte zur Extraktion verwendete Lösungsmittel sind überkritisches CO₂ und Gemische von Wasser mit organischen Lösungsmitteln.

In einer weiteren bevorzugten Ausführungsform betrifft die Erfindung eine Zusammensetzung, die durch die Verwendung von Tectorigenin als Hemmstoff des prostataspezifischen Antigens gekennzeichnet ist. Tectorigenin kann beispielsweise aus den Extrakten von Belamcanda chinensis isoliert werden. Der Stoff ist in seiner Strukturformel in der WO 99/47149 dargestellt. Er weist wie der Extrakt von Belamcanda chinensis PSA-hemmende Wirkung auf und kann somit als das oder eines der Wirkprinzipien in den Extrakten von Belamcanda chinensis angesehen werden. Auch hier können als Ausgangsmaterial für den Exatrkt im wesentlichen alle Bestandteile der Pflanze eingesetzt werden. Dazu gehören Rinde, Äste, Samen, Früchte und Stammholz. Bevorzugt werden zur Herstellung der Extrakte Rhizome, Stengel, Blätter und/oder Blütenblätter der Pflanzen verwendet. Bevorzugte zur Extraktion verwendete Lösungsmittel sind überkritisches CO₂ und Gemische von Wasser mit organischen Lösungsmitteln.

Die vorstehend beschriebenen Ausführungsformen der Erfindung zielen darauf ab, dass diese entweder Extrakte oder bestimmte, namentlich gekennzeichnete Substanzen enthalten. Selbstverständlich sind von der Erfindung auch Ausführungsformen eingeschlossen, in denen sowohl die Reinsubstanzen (oder im wesentlichen reinen/aufgereinigten Substanzen) zusammen mit Extrakten kombiniert werden. Dies wird durch die nachfolgend aufgeführten bevorzugten oder beispielhaften Kombinationsmöglichkeiten veranschaulicht.
1. Silybum marianum + Cimicifuga racemosa + Belamcanda chinensis
2. Silybum marianum + Finasterid + Belamcanda chinensis
3. Silybum marianum + Cimicifuga racemosa + Tectorigenin
4. Silybum marianum + Finasterid + Tectorigenin
5. Silybinin + Finasterid + Tectorigenin
6. Vitex agnus castus + Cimicifuga racemosa + Belamcanda chinensis
7. Vitex agnus castus + Finasterid + Belamcanda chinensis
8. Vitex agnus castus + Cimicifuga racemosa + Tectorigenin
9. Vitex agnus castus + Finasterid + Tectorigenin
10. Apigenin + Finasterid + Tectorigenin

Zusammenfassend kann gesagt werden, dass jeweils an der Stelle eines Extrakts eine Reirisubstanz, die auch als ein wirksamer Bestandteil des Extrakts vorkommt, eingesetzt werden kann. Beispielsweise ist Sylibinin ein Reinstoff, der auch als Bestandteil des Extrakts aus Silybum marianum vorkommt. Die gleiche Beziehung besteht zwischen Apigenin und Vitex agnus castus Extrakt sowie zwischen Tectorigenin und Belamcanda chinensis Extrakt, die jeweils das gleiche Wirkungsprofil aufweisen. Im Falle von Finasterid, Dutasterid und Episterid, die jeweils an Stelle des Extrakts aus Cimicifuga racemosa eingesetzt werden können, gilt jedoch dass die Reinsubstanzen Finasterid, Dutasterid und Episterid einerseits und der Extrakt aus Cimicifuga racemosa andererseits zwar jeweils hemmende Wirkung auf die 5α-Reduktase aufweisen, jedoch sind Finasterid, Dutasterid und Episterid keine nachgewiesenen Bestandteile des Extrakts aus Cimicifuga racemosa. Essentiell ist jedoch immer, dass zumindest ein Vertreter (Reinsubstanz oder Extrakt) eines jeden der drei erfindungswesentlichen Wirkprinzipien in die Zusammensetzung formuliert wird. Entsprechend ist auch eine Zusammensetzung Gegenstand der Erfindung, die - als wirksame Agentien - drei Reinsubstanzen enthält, beispielsweise die oben dargestellte Kombination Nr. 10.

In einer besonders bevorzugten Ausführungsform ist die Zusammensetzung ein Arzneimittel. Bei Formulierung der Zusammensetzung als Arzneimittel ist bevorzugt, dass Reinsubstanzen oder im wesentlichen aufgereinigte Extrakte und nicht nur standardmäßig formulierte Extrakte eingesetzt werden, obwohl letztere Möglichkeit erfindungsgemäß nicht ausgeschlossen ist. Der Begriff "standardmäßig formulierter Extrakt" ist dem Fachmann geläufig und bezieht sich auf die Formulierungsanweisungen wie sie den internationalen Arzneibüchern, beispielsweise dem deutschen oder europäischen Arzneibuch (DAB bzw. EUPHARM) zu entnehmen sind.

Die Arzneimittel enthalten bevorzugt einen pharmazeutisch verträglichen Hilfsstoff.Pharmazeutisch verträgliche Hilfstoffe schließen pharmazeutisch verträgliche Träger oder Exzipienten und pharmazeutisch verträgliche Verdünnungsmittel ein. Beispiele geeigneter Träger sind dem Fachmann bekannt und in intemationalen Arzneibüchern als pharmazeutische Hilfsstoffe monographiert.

Die Arzneimittel können einem Individuum in geeigneter Dosierung verabreicht werden. Die Verabreichung erfolgt bevorzugt oral, parenteral oder subkutan. Besonders bevorzugt ist die orale Verabreichung. Die Höhe der Dosierung wird von dem behandelnden Arzt bestimmt und hängt im wesentlichen von den klinischen Faktoren ab. Diese Faktoren sind in der Medizin und der Wissenschaft bekannt und umfassen beispielsweise die Körpergröße bzw. das Gewicht, die Körperoberfläche, das Alter, das Geschlecht, und den allgemeinen Gesundheitszustand des Patienten, die spezielle zu verabreichende Zusammensetzung, die Behandlungsdauer, die Art der Verabreichung und die eventuelle gleichzeitige Behandlung mit anderen Arzneimittel. Eine typische Dosis kann z.B. in einem Bereich zwischen 0,001 und 5000 mg der Extraktstoffe bzw. der Reinsubstanzen liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Zusammensetzung die Dosis in einem Bereich zwischen 1 mg- und 1000 mg-Einheiten pro Tag befinden. Wird die Zusammensetzung intravenös verabreicht, was nicht bevorzugt empfohlen wird, um die Gefahr einer anaphylaktischen Reaktionen zu minimieren, sollte sich die Dosis in einem Bereich zwischen 1 µg- und 10 mg-Einheiten pro Kilogramm Körpergewicht pro Minute befinden. In jenen Ausführungsformen, in denen Finasterid zum Einsatz gelangt, ist bevorzugt eine Dosis von etwa 1 mg bis etwa 10 mg Finasterid pro Tag, stärker bevorzugt 5 mg pro Tag anzuwenden. In jenen Ausführungsformen, in denen ein Extrakt aus Cimicifuga racemosa zum Einsatz gelangt, ist bevorzugt eine Dosis von etwa etwa 10 mg bis etwa 100 mg pro Tag, stärker bevorzugt von etwa 40 mg bis etwa 60 mg pro Tag anzuwenden. Bevorzugte Dosen in der Maus bzw. im in den Beispielen dargestellten Mausmodell liegen im Bereich zwischen 2 und 3 mg pro Tag.

Besonders bevorzugt ist ferner eine erfindungsgemäße Zusammensetzung, die ein Nahrungsergänzungsmittel oder ein Functional Food mit präventiver und/oder therapeutischer Wirkung ist. Im Falle eines Nahrungsergänzungsmittels oder Functional Food wird bevorzugt auf Extrakte zurückgegriffen. Im Falle eines Nahrungsergänzungsmittels oder Functional Food werden die verschiedenen Komponenten vorzugsweise zusammen in einer Dosis formuliert.

Erfindungsgemäß wird davon ausgegangen, dass die Einnahme und bevorzugt die regelmäßige Einname des erfindungsgemäßen Nahrungsergänzungsmittels zur Prävention von Prostataerkrankungen, wie der benignen Prostatahyperplasie und insbesondere des Prostatakarzinoms dient.

Die Erfindung betrifft ferner die Verwendung eines 5α-Reduktase Hemmstoff, eines Östrogenrezeptor β-Agonisten und eines Hemmstoffes des prostataspezifischen Antigens zur Herstellung eines Arzneimittels zur Prävention oder Behandlung einer Prostataerkrankung.

Bezüglich der bevorzugten Ausführungsformen und der Zubereitung/Formulierung des Arzneimittels und dessen Verabreichung wird auf die im Zusammenhang mit der erfindungsgemäßen Zusammensetzung erörterten Ausführungen verwiesen, die gleichermaßen hier Anwendung finden.

Des weiteren betrifft die Erfindung die Verwendung eines 5α-Reduktase Hemmstoffes, eines Östrogenrezeptor β-Agonisten und eines Hemmstoffes des prostataspezifischen Antigens zur Herstellung eines Nahrungsergänzungsmittels zur Prävention einer Prostataerkrankung.

In bevorzugten Ausführungsformen der erfindungsgemäßen Verwendungsform ist Prostataerkrankung ein Prostata-Karzinom oder eine benigne Prostatahyperplasie.

Schließlich sieht die Erfindung ein Verfahren zur Behandlung eines Patienten mit einer Prostataerkrankung vor, umfassend die gleichzeitige oder sequentielle Verabreichung einer Kombination aus einem 5α-Reduktase Hemmstoff, einem Östrogenrezeptor β-Agonisten und einem Hemmstoff des prostataspezifischen Antigens.

Bezüglich der bevorzugten Ausführungsformen des erfindungsgemäßen Verfahrens wird auch hier auf die vorstehend diskutierten bevorzugten Ausführungsformen im Zusammenhang mit der erfindungsgemäßen Zusammensetzung und der erfindungsgemäßen Verwendung verwiesen. Die dort diskutierten bevorzugten Ausführungsformen treffen *mutatis mutandis* auch für das erfindungsgemäße Behandlungsverfahren zu.

### Die Figuren zeigen:

### Figur 1:

Es ist das normale Gleichgewicht zwischen der proliferationsstimulierenden Wirkung von 5αDHT und der proliferationshemmenden Wirkung von 3βAdiol dargestellt. Da beide Hormone durch die Aktivität der 5α-Reduktase aus Testosteron gebildet werden, führt eine Hemmung der 5α-Reduktase zwar zur Reduktion von 5αDHT und damit zur Reduktion des proliferativen Stimulus, gleichzeitig aber auch zur Reduktion des proliferationshemmenden Einflusses von 3βAdiol. Diese Situation ist durch die im Vergleich zu Fig. 1 a dünneren Pfeile in 1 b dargestellt.

Im Endeffekt führt also eine Hemmung der 5α-Reduktase zu einem neuen Gleichgewicht zwischen Stimulation und Inhibition des Prostatagewebes, sei es hypertroph oder karzinomatös verändert.

Die in Fig. 1a und b dargestellten Zusammenhänge lassen es sinnvoll erscheinen, einen 5α-Reduktasehemmer mit einem ERβ-Agonisten zu kombinieren (Fig. 2). Auf diese neuen therapeutischen Ansätze wird weiter eingegangen.

### Figur 2:

Die gleichzeitige Hemmung der 3βAdiol-Produktion in der Prostata durch Hemmung der 5α-Reduktase kann kompensiert werden, durch die zusätzliche Gabe eines ERβ-Agonisten.

### Figur 3:

Fig. 3a zeigt die deletären Effekte vermehrter PSA-Sekretion der Prostata durch Inaktivierung des IGF1 binding proteins wird intraprostatisch vermehrt IGF1 freigesetzt, welches zur Stimulation des Prostatakarzinomgewebes führt. Das führt zu einem Circulus vitiosus, der durch Hemmung der PSA-Sekretion durchbrochen werden kann (Fig. 3b).

### Figur 4:

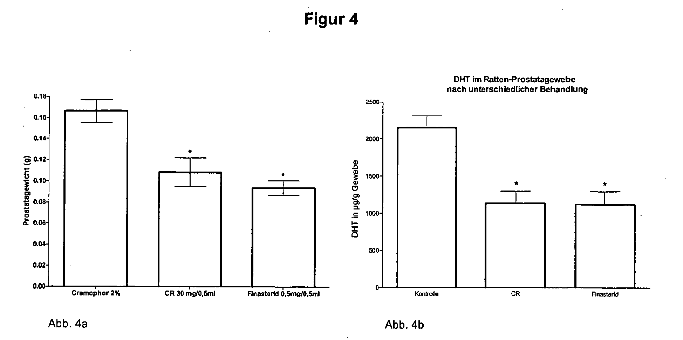

Das Wachstum der Prostatae von präpubertären Ratten kann durch Gabe von Testosteron stark stimuliert werden. Diese Stimulation ist durch Gabe des synthetischen 5α-Reduktasekemmers Finasterid, sowie durch Cimicifuga racemosa Extrakt (CR) signifikant hemmbar (Fig. 4a). Messung von 5α-DHT, dem Produkt der 5α-Reduktase, zeigt unter beiden Behandlungen eine deutliche Reduktion dieses Hormones im vergleich zu den kontrollbehandelten Tieren, das ist ein Beweis für die Hemmung der 5α-Reduktase durch Cimicifuga racemosa Extrakt (Fig. 4b).

### Figur 5:

Das Wachstum von LNCaP-Zellen, wird dosisabhängig und zwar in sehr niedrigen Konzentrationen durch Cimicifuga racemosa Extrakt gehemmt.

### Figur 6:

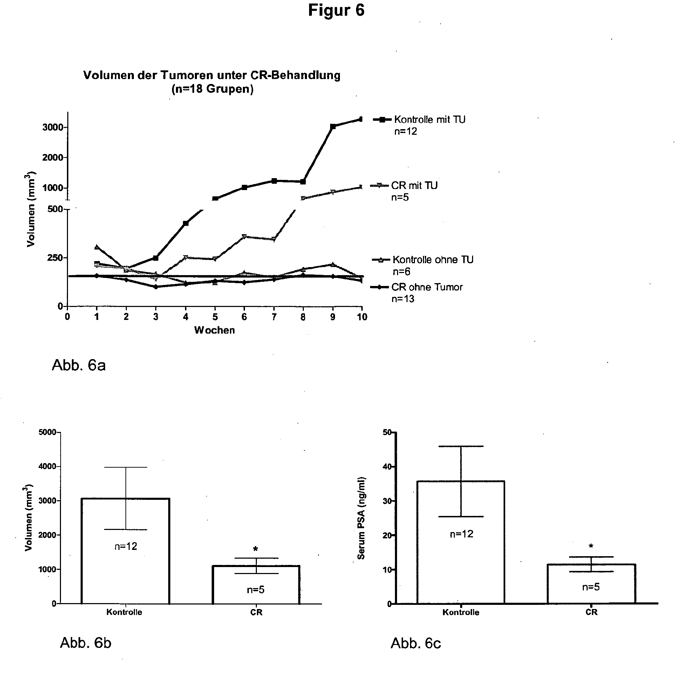

Wirkung von Cimicifuga racemosa Extrakt auf die Angehrate sowie das Wachstum von Prostatakarzinomen in LNCaP inoculierten athymischen nu/nu-Mäusen (Fig. 6a).

Von insgesamt 18 Kontrollen haben 12 einen Tumor bekommen, der innerhalb von 10 Wochen zur Tötung der Tiere führte, da Tiere, die tumorbedingt behindert waren, getötet werden mussten. Die 18 mit Cimicifuga racemosa Extrakt behandelten Tiere entwickelten bis auf 5 überhaupt keine Tumoren. Bei den 5 Tieren, die Tumoren entwickelten, war das Wachstum deutlich verzögert, so dass zum Zeitpunkt der Tötung der Kontrolltiere das Tumorvolumen deutlich niedriger war. Nicht gezeigt werden hier die benigner erscheinenden histologischen Bilder der mit Cimicifuga racemosa Extrakt behandelten Tiere.
Die Tumorvolumina am Ende des Versuchs aller Tiere sind in Fig. 6b gezeigt.
In Fig. 6b und 6c sind die niedrigeren Tumorvolumina und die deutlich niedrigeren Serum-PSA-Werte am Ende des Versuchs der CR-behandelten Tiere im Vergleich zu den Kontrollen gezeigt.

### Figur 7:

Aus Belamcanda chinensis isoliertes Tectorigenin hemmt die PSA-Sekretion von LNCaP-Zellen unter in vitro Bedingungen. Diese Eigenschaft von Tectorigenin findet sich auch im Extrakt aus Belamcanda chinensis (BC) wieder.

### Figur 8:

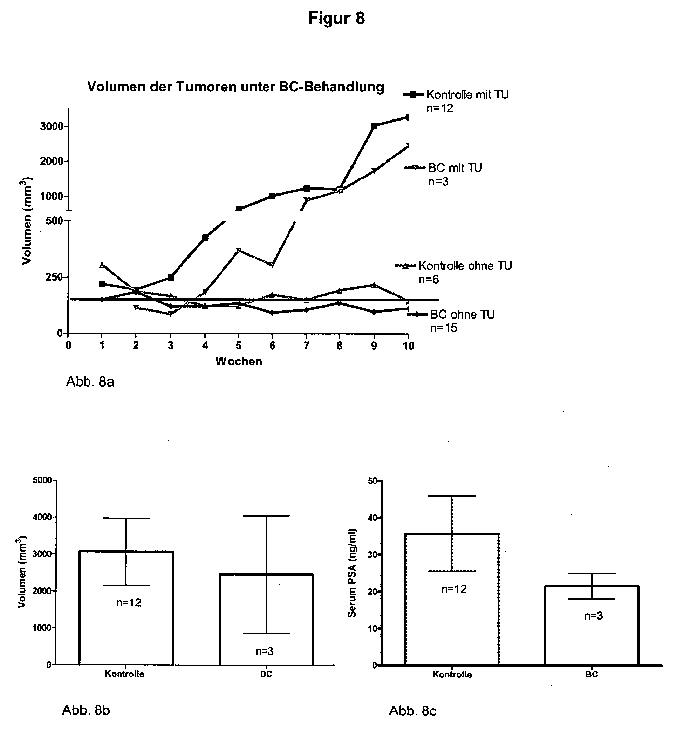

Fig. 8a zeigt eine deutliche Hemmung der Angehrate von LNCaP-induzierten Tumoren in männlichen nu/nu-Mäusen. 3 von 18 BC-behandelten Tieren entwickelten Tumore. Die Mittelwerte der Endvolumina der Tumoren der BC-behandelten Tiere waren nur geringfügig kleiner als die der Kontrollen (Fig. 8b), da sich bei 1 Tier ein extrem großer Tumor entwickelte.
Im Serum der nicht-tumortragenden Tiere ist PSA entweder nicht nachweisbar oder in den wenigen tumortragenden Tieren niedriger als in den Kontrolltieren (Fig. 8c).

### Figur 9:

In Figur 9 ist der Versuch gemacht, die Wirkzusammenhänge der Komponenten der erfindungsgemäßen Zusammensetzung graphisch darzustellen. Eine Kombinationstherapie mit Cimicifuga racemosa Extrakt wird die 5α-Reduktase hemmen, der nachteilige Einfluss der Hemmung der Bildung von 3βAdiol kann durch gleichzeitige Gabe des ERβ-Agonisten in Silybum marianum (oder Agnus castus) kompensiert werden. Eine zusätzliche Therapie mit einem Belamcanda-chinensis-Extrakt hat besonders starke hemmende Wirkungen auf die PSA-Sekretion und wird damit die Bioverfügbarkeit von IGF1 und damit dem proliferationsstimulierenden Effekt dieses Wachstumsfaktors inhibieren. Sowohl der Silybum-marianum- als auch der Belamcanda-chinensis-Extrakt würden die Sekretion von VEGF und damit die Hemmung von Gefäßneubildung bewirken.

Die Beispiele erläutern die Erfindung:

### Beispiel 1: Inhibitorische Wirkung von Cimicifuga racemosa Extrakten auf eine menschliche Prostatazelllinie

Es konnten anhand von Studien mit einer von humanen prostatakarzinomabstammenden Zelllinie (der LNCaP-Zelllinie (ATCC-Nummer CRL-1740)) sowie durch tierexperimentelle Untersuchungen folgende Befunde erhoben werden:
- In dem Cimicifuga racemosa (CR) Extrakt sind starke 5α-reduktasehemmende Prinzipien enthalten.

Diese Aussage wird durch die in Figur 4 dargestellten Ergebnisse belegt.

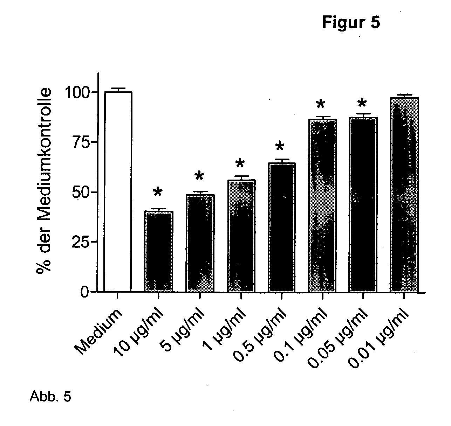

In Fig. 5 ist dargestellt, dass Cimicifuga racemosa Extrakt die Proliferation von LNCaP-Zellen inhibiert.

### Beispiel 2: Inhibitorische Wirkung von Cimicifuga racemosa Extrakten in vivo

Es gibt einen Stamm von Mäusen, der keinen Thymus entwickelt und damit keine T-Zellen produziert. Diese immundefizienten nu/nu-Mäuse eignen sich, um die Eigenschaften von Heterotransplantaten, die normalerweise immunologisch bedingt abgestoßen würden, zu studieren. Deshalb wurden nu/nu-Mäuse subkutan mit LNCaP-Zellen (1 Mio. pro Tier) inoculiert und das Tumorwachstum unter Cimicifuga racemosa Extrakt in zwei Dosen mit dem Futter verabfolgt, studiert. Hierbei kam der wässrig-ethanolische Extrakt aus Cimicifugawurzelstock mit der internen Bezeichnug CR BNO1055 zum Einsatz. Die Fig. 6 zeigt deutlich eine Hemmung der Angehraten von Tumoren, als auch ein verlangsamtes Wachstum der wenigen sich entwickelnden Tumoren im Vergleich zu den nicht CR-behandelten Kontrolltieren.

### Beispiel 3: Wirkung von Belamcanda chinensis Extrakten bzw. von Tectorigenin in vitro und in vivo

Schon früher ist der Nachweis gelungen, dass dem Hauptinhaltstoff aus Extrakten der Mariendistel (Silybum marianum), dem Silybinin (Seidlova-Wuttke et al., loc. cit.) auch eine ERβ-agonistische Wirkung zukommt. Das hat dazu geführt, dass die Wirkung von Silybinin auf LNCaP-Zellwachstum, PSA-Sekretion etc. untersucht wurde, wobei sich herausstellte, dass Silybinin ein reiner ERβ-Agonist ist, der das Wachstum von LNCaP-Zellen, die PSA-Sekretion sowie die Produktion des *vascular endothelial growth factors* (VEGF) hemmt. VEGF wird bei raschem Tumorwachstum vermehrt produziert, die dadurch ermöglichte Kapillarneubildung ist für progressives Tumorwachstum unabdingbar, es ermöglicht also erst das rasche Wachstum von Tumoren. Seine Hemmung hat zur Entwicklung von VEGF-Antagonisten geführt, die sich zurzeit in klinischer Testung zur Hemmung des Tumorwachstums befinden.

Erfindungsgemäß wurden die Wirkungen eines Extraktes aus Belamcanda chinensis und dem daraus isolierten Tectorigenin studiert:

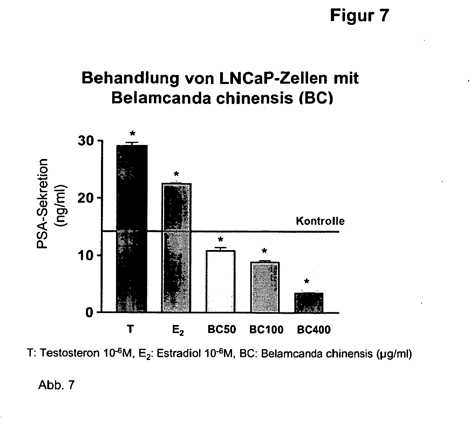

Aus Belamcanda chinensis isoliertes Tectorigenin hemmt die PSA-Sekretion von LNCaP-Zellen unter *in vitro* Bedingungen. Diese Eigenschaften von Tectorigenin finden sich auch im Gesamtextrakt wieder (Fig. 7). Außerdem wird die Genexpression eines Coaktivators des Androgenrezeptors gehemmt, so dass eventuell vorhandene Androgene schwächer durch den Androgenrezeptor wirken können.

Die orale Applikation von Belamcanda chinensis bei LNCaP-zellinoculierten nu/nu-Mäusen führt zu vergleichbarer Hemmung der Tumorangehrate sowie des Tumorwachstums wie unter Cimicifuga racemosa Extrakt (Fig. 8a und b). Wie bei den mit Cimicifuga racemosa Extrakt behandelten Tieren waren im Serum der Belamcanda-chinensis-behandelten Tiere die PSA-Werte deutlich niedriger als bei den Kontrolltieren (Fig. 8c).

## Patentansprüche

1. Zusammensetzung aus Komponenten zur gleichzeitigen oder sequentiellen Verabreichung oder Einnahme, wobei die Komponenten umfassen:
(a) einen 5α-Reduktase Hemmstoff;
(b) einen Östrogenrezeptor β-Agonisten; und
(c) einen Hemmstoff des prostataspezifischen Antigens.

2. Zusammensetzung nach Anspruch 1, wobei als 5α-Reduktase Hemmstoff ein Extrakt aus Cimicifuga racemosa eingesetzt wird.

3. Zusammensetzung nach Anspruch 1, wobei als 5α-Reduktase Hemmstoff Finasterid eingesetzt wird.

4. Zusammensetzung nach einem der Ansprüche 1 bis 3, wobei als Östrogenrezeptor β-Agonist ein Extrakt aus Vitex agnus cactus oder aus Silybum marianum eingesetzt wird.

5. Zusammensetzung nach einem der Ansprüche 1 bis 3, wobei als Östrogenrezeptor β-Agonist Silybinin oder Apigenin eingesetzt wird.

6. Zusammensetzung nach einem der Ansprüche 1 bis 5, wobei als Hemmstoff des prostataspezifischen Antigens ein Extrakt aus Belamcanda chinensis eingesetzt wird.

7. Zusammensetzung nach einem der Ansprüche 1 bis 5, wobei als Hemmstoff des prostataspezifischen Antigens Tectorigenin eingesetzt wird.

8. Zusammensetzung nach einem der Ansprüche 2 bis 7, wobei der Extrakt aus Cimicifuga racemosa ein wässrig-ethanolischer Extrakt aus Cimicifugawurzelstock ist.

9. Zusammensetzung nach einem der Ansprüche 1 bis 8, die ein Arzneimittel ist.

10. Zusammensetzung nach einem der Ansprüche 1 bis 8, die ein Nahrungsergänzungsmittel ist.

11. Verwendung eines 5α-Reduktase Hemmstoff, eines Östrogenrezeptor β-Agonisten und eines Hemmstoffes des prostataspezifischen Antigens zur Herstellung eines Arzneimittels zur Prävention oder Behandlung einer Prostataerkrankung.

12. Verwendung eines 5α-Reduktase Hemmstoff, eines Östrogen rezeptor β-Agonisten und eines Hemmstoffes des prostataspezifischen Antigens zur Herstellung eines Nahrungsergänzungsmittels zur Prävention einer Prostataerkrankung.

13. Verwendung nach Anspruch 11 oder 12, wobei die Prostataerkrankung Prostata-Karzinom ist.

14. Verwendung nach Anspruch 11 oder 12, wobei die Prostataerkrankung benigne Prostatahyperplasie ist.

15. Verfahren zur Behandlung eines Patienten mit einer Prostataerkrankung umfassend die gleichzeitige oder sequentielle Verabreichung einer Kombination aus einem 5α-Reduktase Hemmstoff, einem Östrogenem Rezeptor-Agonsisten und einem Hemmstoff des prostataspezifischen Antigens.
